(19) Europäisches Patentamt
European Patent Office
Office européen des brevets

(11) Veröffentlichungsnummer: **0 175 251**
**B1**

# (12) EUROPÄISCHE PATENTSCHRIFT

(45) Veröffentlichungstag der Patentschrift: **13.07.88**

(51) Int. Cl.⁴: **C 07 C 69/007**, C 11 B 9/00, A 24 B 15/30

(21) Anmeldenummer: **85111360.5**

(22) Anmeldetag: **09.09.85**

(54) **Geschmackstoffkompositionen mit einem Gehalt an Ionylestern, die Ionylester, deren Herstellung und Verwendung.**

(30) Priorität: **21.09.84 CH 4523/84**

(43) Veröffentlichungstag der Anmeldung:
**26.03.86 Patentblatt 86/13**

(45) Bekanntmachung des Hinweises auf die Patenterteilung:
**13.07.88 Patentblatt 88/28**

(84) Benannte Vertragsstaaten:
**CH DE FR GB LI NL**

(56) Entgegenhaltungen:
**ACTA CHEMICA SCANDINAVICA, Band 27, Nr. 6, 1973, Seiten 2107-2114, Kopenhagen, DK.; A.J. AASEN et al.: Tobacco chemistry. 18. absolute configuration of (9R)-9-hydroxy-4,7E-megastigmadien-3-one (3-Oxo-koppa-ionol)**
**HELVETIA CHIMICA ACTA, Band 65, Nr. 6, 1982, Seiten 1927-1928, Basel, CH; S. KATSUMURA et al.: "A New Type of Photorearrangement of Gamma-Hydroxy-Alpha, Beta, Delta, Epsion-dienone"**
**CHEMICAL ABSTRACTS, Band 75, Nr. 23, 6. Dezember 1971, Seiten 49-50, Spalten 1, 2, Zusammenfassungs Nr. 137451r, Columbus, Ohio, US; C.R. ENZELL et al.: "Tobacco Chemistry. 7. Structure and synthesis of 3-oxo-alpha-ionol, a new tobacco constituent"**

(73) Patentinhaber: **L. GIVAUDAN & CIE Société Anonyme, CH-1214 Vernier-Genève (CH)**

(72) Erfinder: **Kaiser, Roman, Weidstrasse 6, CH-8610 Uster (CH)**

(74) Vertreter: **Urech, Peter, Dr. et al, Grenzacherstrasse 124 Postfach 3255, CH-4002 Basel (CH)**

**EP 0 175 251 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Geschmackstoffkompositionen, die durch einen Gehalt an einem Oxo-ionylester der allgemeinen Formel

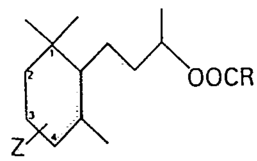

I

worin RCO für einen Acylrest mit 1 – 18 Kohlenstoffatomen steht, eine der gestrichelt gezeichneten Linien im Ring eine zusätzliche Bindung und die gestrichelt gezeichnete Linie in der Seitenkette eine fakultative zusätzliche Bindung bedeutet und Z eine Oxogruppe in α-Stellung zur ringständigen Doppelbindung darstellt, gekennzeichnet sind.

Die Formel I soll sämtliche im Hinblick auf vorhandene cis/trans-Isomerie möglichen geometrischen Isomeren erfassen.

Beispiele von $C_{1-18}$-Acylresten sind Formyl, $C_{2-18}$-Alkanoyl wie Acetyl, Propionyl, Butyroyl, Valeroyl, Isovaleroyl, Stearoyl, $C_{3-18}$-Alkenoyl mit einer oder mehreren Doppelbindungen, wie Crotonoyl, Linoloyl, Linolenoyl, Oleoyl, $C_{7-18}$-Arylalkanoyl, wie Phenylalkanoyl, z. B. Phenylacetyl.

Die bevorzugten Acylreste enthalten bis zu 5 C-Atome.

Bevorzugte Verbindungen I sind insbesondere:
4-Oxo-β-ionylvalerat
4-Oxo-β-ionylisovalerat
4-Oxo-β-ionylbutyrat
4-Oxo-β-ionylacetet
4-Oxo-β-ionylformiat
4-Oxo-β-ionylcrotonat
4-Oxo-dihydro-β-ionylvalerat
4-Oxo-dihydro-β-ionylacetat
3-Oxo-α-ionylvalerat

Die Verbindungen I weisen besondere organoleptische Eigenschaften auf, auf Grund derer sie sich vorzüglich als Geschmackstoffe, insbesondere zur Aromatisierung von Tabakerzeugnissen eignen. Die neuen Geschmackstoffkompositionen eignen sich diesbezüglich insbesondere zur Verstärkung, Verbesserung, Erhöhung oder Modifizierung der organoleptischen Eigenschaften von Tabakerzeugnissen.

Die Erfindung betrifft auch die Verwendung der Verbindungen I als Geschmackstoffe, insbesondere als Geschmackstoffe zur Tabakaromatisierung und ein Verfahren zur Verstärkung, Verbesserung, Erhöhung oder Modifizierung der organoleptischen Eigenschaften von Tabakerzeugnissen mittels der Verbindungen I.

«Tabakerzeugnis» ist eine in der Branche übliche, allgemeine Bezeichnung, die aber nicht nur Tabak [-z. B. Burley-, Maryland-, Virginia-, Kentukky-, orientalische Sorten oder Mischungen davon] selbst umfasst, sondern auch Tabaknebenprodukte, wie rekonstituierte und homogenisierte Blätter und Stengel, Tabaksurrogate (z. B. Salat- und Kohlblätter, etc.), Materialien, die bei der Tabakverarbeitung gebraucht werden, wie Papier, Filter. Unter die Bezeichnung «Tabakerzeugnis» fallen Zigarettentabak, Zigarrentabak, Kautabak und Pfeifentabak.

Der Zusatz eines Oxo-ionylesters der Formel I beispielsweise zu einer Tabakmischung kann sich je nach Art des verwendeten Esters I beim Verrauchen verschiedenartig auf die organoleptischen Eigenschaften auswirken. So zeigt z. B. das (Rauch)Aroma von mit 4-Oxo-β-ionylvalerat behandelten Zigaretten die typischen Aspekte von dunklem Tabak und wirkt im gesamten wesentlich abgerundeter und voller.

Bei Verwendung von 4-Oxo-β-ionylacetet können insbesondere eine allgemeine Verstärkung des Tabakcharakters und zusätzliches Auftreten von blumigen, würzigen und teeartigen Aspekten festgestellt werden. Andere Verbindungen der Formel I liefern beim Verrauchen wiederum wünschenswerte neuartige, caramelartige, fruchtige, holzige oder süssblumige Aspekte oder bereichern den Gesamteindruck durch das Auftreten eines sehr erwünschten «Salivating-Effektes».

Allgemein kann gesagt werden, dass der Zusatz eines Oxo-ionylesters der Formel I zu einem Tabakerzeugnis dessen Rauchcharakteristik deutlich verbessert, insbesondere den allgemeinen Tabakeindruck verstärkt, das gesamte Aroma abgerundeter und voller erscheinen lässt und gegebenenfalls durch die oben aufgezählten organoleptischen Aspekte zusätzlich bereichert.

Die Tatsache, dass die Verbindungen der allgemeinen Formel I recht schwerflüchtig sind und die aromaaktiven Verbindungen insbesondere beim Verrauchen entstehen. Das bringt erhebliche Vorteile bezüglich Aromakonstanz und Stabilität des Fertigproduktes mit sich. Die üblicherweise zur Tabakaromatisierung verwendeten Verbindungen sind nämlich häufig relativ leichtflüchtig, so dass beim Lagern der aromatisierten Tabakprodukte im Verlaufe der Zeit bisher immer ein gewisser Aromaverlust in Kauf genommen werden musste. Dieser Nachteil kann durch Verwendung von Verbindungen der Formel I weitgehend verhindert werden.

Die Menge des Esters der Formel I, die zweckmässigerweise zugesetzt wird, kann von verschiedenen Faktoren, einschliesslich der gewünschten Wirkung, der Art und der Menge anderer, gleichzeitig verwendeter Zusätze und/oder der persönlichen Vorliebe des Aromatikers, abhängen. Schon Mengen von 10 ppm, bezogen auf das Gewicht des Tabaks, erweisen sich als wirksam, während aber auch Mengen von 10 000 ppm noch verwendbar sind. Besonders bevorzugt werden jedoch Mengen von 50 ppm bis 2 000 ppm eingesetzt.

Die oben vorgeschlagenen Grenzen sollen selbstverständlich nur die bevorzugten Mengen andeuten; diese sind jedoch auch von der Geschicklichkeit des Aromatikers und der Wirkung, die er erzielen will, abhängig.

Die Herstellung von Geschmackstoffkompositionen zur Tabakaromatisierung kann durch einfaches Auflösen von Verbindungen I in einem geeigneten Lösungsmittel, insbesondere einem polaren Lösungsmittel, beispielsweise Alkohol, Propylenglykol, gegebenenfalls unter Zusatz von weiteren (Riech- und/oder) Aromastoffen und von Wasser geschehen, wobei die Konzentration der Verbindung I z. B. 0,5 bis 50% betragen kann.

Die Verbindungen I können dem Tabakprodukt (Zigaretten), beispielsweise in Form obiger Geschmackstoffkompositionen nach den, dem Fachmann bekannten Methoden, z. B. Versprühen, Zerstäuben, Eintauchen, Überziehen, zugesetzt oder beigemischt werden.

Die Verbindungen der Formel I sind, mit Ausnahme der Acetate, neue Verbindungen. Die neuen Verbindungen der Formel I bilden deshalb ebenfalls Gegenstand der vorliegenden Erfindung.

Die Verbindungen I können auf an sich bekannte Art und Weise erhalten werden, also durch Veresterung von Oxo-ionolen der Formel

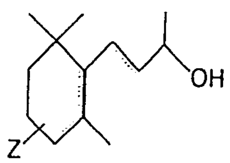

II

worin eine der gestrichelt gezeichneten Linien im Ring eine zusätzliche Bindung und die gestrichelt gezeichnete Linie in der Seitenkette eine fakultative zusätzliche Bindung darstellen, und Z eine Oxogruppe in α-Stellung zur ringständigen Doppelbindung darstellt.

Die Veresterung der Oxo-ionole II kann in an sich bekannter Weise unter Verwendung üblicher Acylierungsmittel, also z. B. Acylhalogeniden, insbesondere dem Chlorid, oder Säureanhydriden durchgeführt werden. Bevorzugt ist die Arbeitsweise unter Verwendung der Säureanhydride. Man arbeitet zweckmässigerweise in Anwesenheit einer Base, z. B. eines organischen Amins wie Pyridin oder Dimethylanilin, und gegebenenfalls unter Verwendung eines inerten Lösungsmittels, z. B. eines Kohlenwasserstoffes oder eines Äthers, z. B. Hexan, Cyclohexan, Toluol, Diäthyläther. Ein geeigneter Temperaturbereich ist derjenige von etwa 20 °C – 80 °C; siehe auch Organikum, Org. chem. Grundpraktikum VEB Deutscher Verlag der Wissenschaften, Berlin (1977), Seiten 499 ff.

Beispiel 1

3,26 g (0,032 Mol) Essigsäureanhydrid werden unter Rühren mit 1,83 g (0,040 Mol) Ameisensäure versetzt, das Gemisch wird während 30 Minuten bei 60 °C gehalten, dann auf 0 °C abgekühlt, 5,00 g (0,024 Mol) 4-Oxo-β-ionol werden zugetropft, und anschliessend wird während 16 Stunden bei 0 °C und 1 Stunde bei Raumtemperatur gerührt. Zur Aufarbeitung wird mit 50 ml Hexan versetzt, die organische Phase wird je zweimal mit Wasser, Sodalösung und wieder mit Wasser gewaschen, mit Natruimsulfat getrocknet und die filtrierte Lösung eingeengt. Die Kugelrohrdestillation des Rohproduktes (5,2) ergibt 4,5 g >93%-iges 4-Oxo-β-ionylformiat. Sdp.ca. 110 °C/0,05 mmHg.

IR: 1720, 1667, 1600, 1175, 1040, 970 $cm^{-1}$.

NMR: 1,13 (2s, 6H); 1.42 (d, J = 6.5, 3H); 1.80 (s, 3H); 1.87 (m, 2H); 2.47 (m, 2H); 5.58 (dxd, $J_1$~16 Hz, $J_2$~6Hz, 1H); ca. 5.55 (m, 1H); 6.30 (dxm, 1H); 8.12 (s, 1H).

MS: 236 ($M^+$, 2), 208 (2), 190 (40), 175 (14). 163 (25), 147 (16), 134 (47), 119 (31), 105 (21), 91 (24), 77 (15), 69 (11), 55 (24), 43 (100).

Zur organoleptischen Beurteilung wird eine 5%-ige Lösung des Esters in 95%-igem Alkohol hergestellt und davon mit Hilfe einer Injektionsnadel 10 µl gleichmässig über die gesamte Länge der Testzigaretten (American blend, bestehend aus Virginia Tabak, Orient Tabak und Burley Tabak) verteilt und anschliessend die so behandelten Zigaretten während 24 Stunden rekonditioniert. Zur Herstelllung der entsprechenden Kontrollzigaretten wird das gleiche Verfahren mit 10 µl 95%-igem Alkohol wiederholt. Das Expertenpanel bezeichnet das Raucharoma der Testzigaretten als wesentlich typischer und ausgewogener tabakartig; zusätzlich wurden süss-blumige Nuancen nachgewiesen.

Beispiel 2

Eine Lösung von 35,4 g (0,17 Mol) 4-Oxo-β-ionol in 20,1 g (0,26 Mol) Pyridin wird bei Raumtemperatur im Verlaufe von 5 Minuten mit 20,8 g (0,20 Mol) Essigsäureanhydrid versetzt und anschliessend das Reaktionsgemisch während 4 Stunden bei 50 °C gerührt. Zur Aufarbeitung wird auf 20 °C gekühlt, mit 200 ml Hexan versetzt, die organische Phase je zweimal mit Wasser, verdünnter Salzsäure, Wasser, Natriumbicarbonatlösung und wieder mit Wasser gewaschen, mit Natriumsulfat getrocknet und die filtrierte Lösung eingeengt.

Die Destillation des Rohproduktes über eine 15 cm-Widmerkolonne ergibt 31,8 g (75%) >93%-iges 4-Oxo-β-ionylacetat. Sdp. 117 °C/0,01 mmHg.

IR: 1740, 1670, 1600, 1230, 1095, 1045, 970, 950 $cm^{-1}$.

NMR: 1.17 (2s, 6H); 1.40 (d, J~6.5, 3H); 1.82 (s, 3H); 1.90 (m, 2H); 2.08 (s, 3H); 1.90 (m, 2H); 2.08 (s, 3H); 2.47 (m, 2H); 5.58 (dxd, $J_1$~16 Hz, $J_2$~6 Hz, 1H); 5.55 (m, 1H); 6.28 (dxm, 1H).

MS: 250 ($^+$, 6), 208 (33), 190 (10), 175 (12), 165 (43), 147 (12), 134 (34), 119 (19), 105 (16), 91 (23), 77 (14), 65 (9), 55 (17), 43 (100).

Das Expertenpanel bezeichnete das Raucharoma der analog Beispiel 1 hergestellten Testzigaretten als eindeutig tabakartiger, daneben auch würziger und leicht blumiger als jenes der Kontrollzigaretten.

Beispiel 3

Eine Lösung von 31,2 g (0,15 Mol) 4-Oxo-β-ionol in 23,7 g (0,30 Mol) Pyridin wird bei Raumtemperatur im Verlauf von 5 Minuten 19,5 g (0,15 Mol) Propionsäureanhydrid versetzt und anschliessend das Reaktionsgemisch während 4 Stunden bei 50 °C gerührt. Das übliche Aufarbeiten (siehe 4-Oxo-β-ionylacetat) liefert 36,8 g Rohprodukt, aus denen durch Destillation über eine 10 cm-Widmerkolonne 28,9 g (73%) 4-Oxo-β-ionylpropionat

(Reinheit >93%) gewonnen werden. Sdp. 132 °C/ 0,05 mmHg.

IR: 1735, 1665, 1600, 1197, 1090, 1050, 980.

MS: 264 ($M^+$, 3); 208 (33), 190 (5), 175 (10), 165 (40), 147 (12), 134 (30), 119 (21), 105 (21), 91 (27), 77 (20), 69 (16), 57 (100), 43 (68).

Das Expertenpanel bezeichnete das Raucharoma der analog Beispiel 1 hergestellten Testzigaretten als süsser, heuartiger und gesamthaft betrachtet den Tabakcharakter wesentlich besser unterstreichend und abgerundeter als jenes der Kontrollzigaretten.

Beispiel 4

Auf analogem Wege werden aus 10,40 g (0,05 Mol) 4-Oxo-β-ionol in 7,90 g (0,1 Mol) Pyridin und 9,48 g (0,06 Mol) Buttersäureanhydrid 14,2 g Rohprodukt erhalten, aus denen durch Destillation über eine 10 cm-Widmerkolonne 9,9 g (71%) 4-Oxo-β-ionylbutyrat (Reinheit >93%) gewonnen werden können. Sdp. 140 °C/0,05 mmHg.

IR: 1735, 1665, 1600, 1255, 1185, 1100, 1050, 975 $cm^{-1}$.

MS: 278 ($M^+$, 1), 208 (20), 190 (3), 175 (6), 165 (17), 147 (6), 134 (18), 119 (10), 105 (9), 91 (12), 77 (9), 71 (29), 55 (17), 43 (100).

Das Expertenpanel bezeichnete das Raucharoma der analog Beispiel 1 hergestellten Testzigaretten als wesentlich holziger, grüner, fruchtiger als jenes der Kontrollzigaretten.

Beispiel 5

Auf analogem Wege werden aus 31,2 g (0,15 Mol) 4-Oxo-β-ionol in 23,7 g (0,30 Mol) Pyridin und 27,9 g (0,15 Mol) Valeriansäureanhydrid 39,5 g Rohprodukt erhalten, aus denen durch Destillation über eine 10 cm-Widmerkolonne 30,2 g (69%) 4-Oxo-β-ionylvalerat (Reinheit >92%) gewonnen werden können. Sdp. 145–147 °C/ 0,05 mmHg.

IR: 1735, 1665, 1600, 1255, 1180, 1100, 1050, 980 $cm^{-1}$.

MS: 292 ($M^+$, 1), 208 (49), 190 (5), 175 (11), 165 (44), 147 (13), 134 (38), 119 (20), 109 (20), 91 (28), 85 (31), 77 (19), 69 (21), 57 (89), 43 (86), 41 (100).

Das Expertenpanel bezeichnete das Raucharoma der analog Beispiel 1 hergestellten Testzigaretten als eindeutiger an dunklen Tabak erinnernd und abgerundeter als jenes der Kontrollzigaretten.

Beispiel 6

Auf analogem Wege werden aus 31,2 g (0,15 Mol) 4-Oxo-β-ionol in 23,7 g (0,30 Mol) Pyridin und 23,1 g (0,15 Mol) Crotonsäureanhydrid 41,0 g Rohprodukt erhalten, aus denen durch Destillation über eine 10 cm-Widmerkolonne 30,3 g (73%) 4-Oxo-β-ionylcrotonat (Reinheit >92%) gewonnen werden können. Sdp. 140 – 141 °C/0,05 mmHg.

IR: 1718, 1665, 1600, 1298, 1265, 1188, 1105, 1050, 980 $cm^{-1}$.

MS: 276 ($M^+$, 2), 207 (8), 190 (1), 175 (3), 165 (5), 147 (4), 134 (10), 119 (7), 105 (8), 91 (10), 77 (8), 69 (100), 55 (12), 41 (55).

Das Expertenpanel bezeichnete das Raucharoma der analog Beispiel 1 hergestellten Testzigaretten als eindeutig süsser, caramelartiger und heuartiger als jenes der Kontrollzigaretten.

Beispiel 7

Eine Lösung von 5,00 g (0,024 Mol) 4-Oxo-β-ionol und 2,29 g (0,029 Mol) Pyridin in 25 ml Cyclohexan wird im Verlaufe von 10 Minuten mit einer Lösung von 3,71 g (0,024 Mol) Phenylacetylchlorid in 5 ml Cyclohexan versetzt. Dabei steigt die Temperatur des Reaktionsgemisches auf 35°C. Anschliessend wird während 4 Stunden bei Raumtemperatur gerührt, dann mit 50 ml Äther verdünnt, die organische Phase je zweimal mit verdünnter Salzsäure, Wasser, Sodalösung und wieder mit Wasser gewaschen, getrocknet und eingeengt. Nach zweistündigem Evakuieren am Hochvakuum verbleiben 7,1 g (91%) 4-Oxo-β-ionylphenylacetat (Reinheit >90%).

IR: 1735, 1665, 1600, 1500, 1250, 1140, 1040, 965 $cm^{-1}$.

MS: 326 ($M^+$, 8), 208 (78), 193 (16), 190 (15), 175 (14), 165 (96), 147 (18), 137 (28), 134 (38), 121 (30), 109 (30), 91 (100), 77 (20), 69 (23), 55 (35), 43 (83).

Das Expertenpanel bezeichnete das Raucharoma der analog Beispiel 1 hergestellten Testzigaretten als eindeutig heuartiger als jenes der Kontrollzigaretten.

Beispiele 8

Eine Lösung von 5,00 g (0,024 Mol) 4-Oxo-β-ionol und 2,29 g (0,029 Mol) Pyridin in 30 ml Cyclohexan wird im Verlaufe von 10 Minuten mit einer Lösung von 7,11 g (ca. 0,024 Mol) eines Gemisches, bestehend aus 57% Linolsäurechlorid, 14% Linolsäurechlorid, 18% Oelsäurechlorid, 3% Stearinsäurechlorid und 7% Palmitinsäurechlorid in 10 ml Cyclohexan versetzt. Dabei steigt die Temperatur des Reaktionsgemisches auf 40 °C. Anschliessend wird während 4 Stunden bei Raumtemperatur gerührt, dann mit 50 ml Äther verdünnt, die organische Phase je zweimal mit verdünnter Salzsäure, Wasser, Sodalösung und wieder mit Wasser gewaschen, getrocknet und eingeengt.

Nach zweistündigem Evakuieren am Hochvakuum verbleiben 9,7 g (86%) Produkt, welches ein Gemisch von $C_{18}$- und $C_{16}$-Fettsäureester des 4-Oxo-β-ionols darstellt, und zwar aus ca. 57% 4-Oxo-β-ionyllinolenat, 14% -linoleat, 18% -oleat, 3% -stearat und 7% -palmitat besteht.

IR: 1753, 1665, 1600, 1245, 1175, 1095, 1045, 968, 725 $cm^{-1}$.

MS: 277 (24), 208 (59), 192 (58), 191 (36), 175 (11), 165 (22), 147 (18), 134 (20), 121 (27), 109 (22), 95 (38), 79 (48), 67 (58), 55 (72), 43 (64).

Das Expertenpanel bezeichnete das Raucharoma der analog Beispiel 1 hergestellten Testzigaretten als klarerweise milder und abgerundeter als jenes der Kontrollzigaretten. Zudem war ein eindeutiger «Salivating»-Effekt festzustellen.

Das oben verwendete Säurechloridgemisch wird durch übliche Umsetzung des entsprechenden, durch Verseifung von Leinöl mit einer Base erhältlichen Säuregemisches mit Thionylchlorid erhalten.

Beispiel 9

Analog Beispiel 1 werden aus 5,00 g (0,024 Mol) 4-Oxodihydro-β-ionol 5,1 g Rohprodukt erhalten, aus dem sich durch Kugelrohrdestillation 4,2 g (73,7%) >93%-iges 4-Oxodihydro-β-ionylformiat gewinnen lassen. Sdp.ca. 112 °C/0,05 mmHg.

IR: 1720, 1667, 1610 1182 $cm^{-1}$.

NMR: 1.20 (2s, 6H); 1.37 (d, J~6.5, 3H); 1.80 (s, 3H); 1.60 – 2.70 (m, 8H); 5.12 (m, J~6.5, 1H); 8.08 (s, 1H).

MS: 238 ($M^+$, 2), 192 (28), 177 (15), 163 (27), 149 (15), 137 (51), 135 (47), 121 (43), 109 (55), 93 (31), 81 (28), 67 (36), 55 (66), 43 (100).

Das Expertenpanel bezeichnete das Raucharoma der analog Beispiel 1 hergestellten Testzigaretten als eindeutig tabakartiger, heuartiger als dasjenige der Kontrollzigaretten.

Beispiel 10

Analog Beispiel 2 werden durch Reaktion von 42,3 g (0,20 Mol) 4-Oxo-dihydro-β-ionol in 35,4 g (0,45 Mol) Pyridin mit 30,5 g (0,30 Mol) Essigsäureanhydrid 47,0 g Rohprodukt erhalten, aus denen sich durch Destillation über eine 15 cm-Widmerkolonne 40,3 g (80%) über 93%-iges 4-Oxo-dihydroβ-ionylacetat gewinnen lassen. Sdp. 120°C/ 0,1 mmHg.

IR: 1740, 1670, 1610, 1240, 1200, 1134, 1076, 1022, 958, 948 $cm^{-1}$.

NMR: 1.12 (2s, 6H); 1.22 (d, J~6.5, 3H); 1.72 (s, 3H); 1.55 – 2.55 (m, 8H); 2.02 (s, 3H); 4.91 (m, J~6.5, 1H).

MS: 252 ($M^+$, 1), 192 (100), 177 (34), 163 (93), 149 (19), 135 (49), 121 (60), 107 (24), 93 (19), 55 (19), 43 (48).

Das Expertenpanel bezeichnete das Raucharoma der analog Beispiel 1 hergestellten Testzigaretten als eindeutig tabakartiger, heuartiger und süsser als dasjenige der Kontrollzigaretten. Zudem war ein eindeutiger «Salivating-Effekt» festzustellen.

Beispiel 11

Analog Beispiel 3 werden durch Reaktion von 10,40 g (0,05 Mol) 4-Oxo-dihydro-β-ionol in 7,90 g (0,1 Mol) Pyridin mit 7,80 g (0,06 Mol) Propionsäureanhydrid 12,5 g Rohprodukt erhalten, aus denen sich durch Destillation über eine 10 cm-Widmerkolonne 9,04 g (68%) über 92%-iges 4-Oxo-dihydro-β-ionylpropionat gewinnen lassen. Sdp. 127 °C/0,05 mmHg.

IR: 1735, 1665, 1610, 1200, 1140, 1095, 1030 $cm^{-1}$.

MS: 266 ($M^+$, <1), 192 (75), 177 (30), 163 (65), 149 (18), 135 (38), 121 (65), 107 (26), 93 (25), 79 (27), 67 (30), 57 (100), 55 (52), 43 (62), 41 (64).

Das Expertenpanel bezeichnete das Raucharoma der analog Beispiel 1 hergestelltenTestzigaretten als klarerweise süsser, heuartiger und, gesamthaft betrachtet, als abgerundeter als das Aroma der Kontrollzigaretten.

Beispiel 12

Analog Beispiel 4 werden durch Reaktion von 10,40 g (0,05 Mol) 4-Oxo-dihydro-β-ionol in 7,90 g (0,10 Mol) Pyridin mit 9,48 g (0,06 Mol) Buttersäureanhydrid 14,0 g Rohprodukt erhalten, aus denen sich durch Destillation über eine 10 cm-Widmerkolonne 10,5 g (75%) über 92%-iges 4-Oxo-dihydro-β-ionylbutyrat gewinnen lassen. Sdp. 134 – 136 °C/ 0,05 mmHg.

IR: 1735, 1665, 1610, 1260, 1195, 1140 1095 $cm^{-1}$.

MS: 280 ($M^+$, <1), 192 (60), 177 (24), 163 (51), 149 (14), 135 (25), 121 (43), 107 (17), 93 (17), 79 (16), 71 (28), 55 (33), 43 (100), 41 (57).

Das Expertenpanel bezeichnete das Raucharoma der analog Beispiel 1 hergestellten Testzigaretten als fruchtiger und holziger als dasjenige der Kontrollzigaretten.

Beispiel 13

Analog Beispiel 5 werden aus 10,40 g (0,05 Mol) 4-Oxo-dihydro-β-ionol, 7,90 g (0,10 Mol) Pyridin und 11,10 g (0,06 Mol) Valeriansäureanhydrid 14,5 g Rohprodukt erhalten, aus denen sich durch Destillation 10,3 g (70%) über 90%-iges 4-Oxo-dihydro-β-ionylvalerat gewinnen lassen. Sdp. 138 – 139 °C/0,05 mmHg.

IR: 1735, 1665, 1610, 1258, 1185, 1140, 1120, 1098 $cm^{-1}$.

MS: 294 ($M^+$, <1), 192 (100), 177 (37), 163 (89), 149 (21), 135 (39), 121 (68), 107 (25), 93 (25), 85 (21), 79 (25), 67 (29), 57 (64), 55 (62), 43 (65), 41 (92).

Das Expertenpanel bezeichnete das Raucharoma der analog Beispiel 1 hergestellten Testzigaretten als eindeutig tabakartiger, blumiger, süsser, abgerundeter als jenes der Kontrollzigaretten.

Beispiel 14

Analog Beispiel 6 werden aus 10,4 g (0,05 Mol) Dihydro-β-ionol, 7,90 g (0,10 Mol) Pyridin und 9,24 g (0,06 Mol) Crotonsäureanhydrid 14,5 g Rohprodukt erhalten, aus denen sich durch Destillation über eine 10 cm-Widmerkolonne 9,4 g (68%) >90%-iges 4-Oxo-dihydro-β-ionylcrotonat gewinnen lassen. Sdp. 135 – 137 °C/0,05 mmHg.

IR: 1718, 1665, 1610, 1275, 1195, 1140, 1110, 1115, 980 $cm^{-1}$.

MS: 278 ($M^+$, <1), 192 (49), 177 (20), 163 (40), 149 (12), 135 (23), 121 (51), 107 (21), 93 (22), 79 (22), 69 (100), 55 (38), 43 (44), 41 (85).

Das Expertenpanel bezeichnete das Raucharoma der analog Beispiel 1 hergestellten Testzigaretten als eindeutig würziger und caramelartiger als dasjenige der Kontrollzigaretten.

Beispiel 15

Analog Beispiel 7 werden aus 5,0 g (0,024 Mol) 4-Oxo-dihydro-β-ionol, 2,29 g (0,029 Mol) Pyridin und 3,71 g (0,024 Mol) Phenylacetylchlorid unter Verwendung von Cyclohexan als Lösungsmittel 6,13 g (78%) ca. 90%-iges 4-Oxo-dihydro-β-ionylphenylacetat erhalten.

IR: 1735, 1665, 1605, 1495, 1250, 1130, 1075, 1030, 1010, 960, 765, 720, 700 $cm^{-1}$.

MS: 328 ($M^+$, 2), 192 (100), 177 (24), 163 (67), 149 (14), 137 (30), 135 (33), 121 (43), 109 (21), 91 (92), 81 (16), 67 (17), 55 (29), 43 (24).

Das Expertenpanel bezeichnete das Raucharoma der analog Beispiel 1 hergestellten Testzigaretten als eindeutig heuartiger und süsser als jenes der Kontrollzigaretten.

Beispiel 16

Analog Beispiel 8 werden aus 5,0 g (0,024 Mol) 4-Oxo-dihydro-β-ionol, 2,29 g (0,029 Mol) Pyridin und 7,11 g (ca.0,024 Mol) des Säurechlorid-Gemisches des Beispiels 8 unter Verwendung von Cyclohexan als Lösungsmittel 10,5 g (93%) Produkt erhalten, welches sich aus ca. 57% 4-Oxo-dihydro-β-ionyllinolenat, 14%-linoleat, 18%-oleat, 3% -stearat und 7% -palmitat zusammensetzt.

IR: 1735, 1665, 1610, 1245, 1180, 1130, 1080, 1030, 720 $cm^{-1}$.

MS: 470 ($M^+$, 4), 277 (15), 192 (100), 177 (17), 163 (31), 150 (18), 137 (36), 135 (24), 121 (31), 109 (27), 95 (27), 81 (31), 67 (40), 55 (54), 43 (49).

Das Expertenpanel bezeichnete das Raucharoma der analog Beispiel 1 hergestellten Testzigaretten als eindeutig milder als jenes der Kontrollzigaretten, wobei insbesondere der allgemeine Tabakeindruck im gegenständlichen Fall verstärkt zur Geltung kam. Zudem wurde ein ausgesprochener «Salivating-Effekt» festgestellt.

Beispiel 17

Analog Beispiel 1 werden aus 1,50 g (7,2 mMol) 3-Oxo-α-ionol 1,25 g Rohprodukt erhalten, aus dem sich durch Kugelrohrdestillation 1,00 g >90%-iges 3-Oxo-α-ionylformiat gewinnen lassen. Sdp.ca. 110 °C/0,05 mmHg.

IR: 1720, 1665, 1630, 1250, 1180, 1040, 980, 832 $cm^{-1}$.

MS: 236 ($M^+$, <1), 190 (7), 180 (11), 175 (3), 134 (100), 119 (14), 108 (99), 91 (49), 79 (19), 65 (11), 55 (18), 43 (61).

Das Expertenpanel bezeichnete das Raucharoma der analog Beispiel 1 hergestellten Testzigaretten als eindeutig tabakartiger als das der Kontrollzigaretten.

Beispiel 18

Analog Beispiel 2 werden aus 4,16 g (0,020 Mol) 3-Oxo-α-ionol, 3,16 g (0,040 Mol) Pyridin und 3,00 g (0,029 Mol) Essigsäureanhydrid 4,20 g Rohprodukt erhalten, aus denen sich durch Kugelrohrdestillation 3,50 g (70%) >93%-iges 3-Oxo-α--ionylacetat gewinnen lassen. Sdp.ca. 115–120 °C/0,05 mmHg.

IR: 1735, 1665, 1630, 1240, 1150, 1045, 980, 950, 912, 835 $cm^{-1}$.

NMR: 0.96 und 1.03 (je s, je 3H); 1.32 (d, J = 6.5, 3H); 1.90 (s, 3H); 2.03 (s, 3H); 2.20 (d, J~5.5, 2H); 2.50 (m, 1H); 5.1–5.7 (2xm, 3H); 5.90 (s, 1H).

MS: 250 ($M^+$, >1), 190 (9), 175 (6), 148 (5), 134 (50), 119 (9), 108 (70), 91 (25), 87 (8), 79 (10), 65 (5), 55 (8), 43 (100).

Das Expertenpanel bezeichnete das Raucharoma der analog Beispiel 1 hergestellten Testzigaretten als wesentlich tabakartiger als dasjenige der Kontrollzigaretten, wobei im gegenständlichen Fall insbesondere eine (gleichmässige) Verstärkung des Tabakeindruckes festgestellt wurde.

Beispiel 19

Analog Beispiel 4 werden aus 8,00 g (0,038 Mol) 3-Oxo-α-ionol, 6,00 g (0,075 Mol) Pyridin und 7,20 g (0,045 Mol) Buttersäureanhydrid 9,5 g Rohprodukt erhalten, aus denen sich durch Destillation über eine 10 cm-Widmerkolonne 7,2 g (68%) >93%-iges 3-Oxo-α-ionylbutyrat gewinnen lassen. Sdp. 125 – 127 °C/mmHg.

IR: 1730, 1670, 1630, 1250, 1185, 1095, 1045, 978 $cm^{-1}$.

MS: 278 ($M^+$, 1), 208 (8), 207 (9), 190 (13), 175 (7), 151 (6), 138 (23), 134 (64), 108 (65), 91 (31), 79 (12), 71 (86), 43 (100).

Das Expertenpanel bezeichnete das Raucharoma der analog Beispiel 1 hergestellten Testzigaretten als eindeutig fruchtiger als im Falle des Aromas der Kontrollzigaretten.

Beispiel 20

Analog Beispiel 5 werden 8,00 g (0,038 Mol) 3-Oxo-α-ionol, 6,00 g (0,075 Mol) Pyridin und 8,38 g (0,045 Mol) Valeriansäureanhydrid 10,1 g Rohprodukt erhalten, aus denen sich durch Destillation über eine 5 cm-Widmerkolonne 7,9 g(71%) >90%-iges 3-Oxo-α-ionylvalerat gewinnen lassen. Sdp. 130 °C/10,1 mmHg.

IR: 1730, 1670, 1630, 1250, 1178, 1045, 980 $cm^{-1}$.

MS: 292 ($M^+$, 2), 236 (3), 222 (5), 207 (18), 190 (20), 175 (11), 148 (7), 134 (99), 108 (96), 91 (54), 85 (100), 79 (20), 43 (50).

Das Expertenpanel bezeichnete das Raucharoma der analog Beispiel 1 hergestellten Testzigaretten als klarerweise überlegen, insbesondere mehr an dunklen Tabak erinnernd. Zudem wurde eine allgemeine Verstärkung des Tabakeindruckes festgestellt.

Beispiel 21

Analog Beispiel 6 werden aus 4,16 g (0,020 Mol) 3-Oxo-α-ionol, 3,6 g (0,040 Mol) Pyridin und 4,62 g (0,030 Mol) Crotonsäureanhydrid 4,5 g Rohprodukt erhalten, aus denen sich durch Kugelrohrdestillation 3,8 g (69%) ca. 90%-iges 3-Oxo-α-ionylcrotonat gewinnen lassen. Sdp. 130 °C/0,1 mmHg.

IR: 1720, 1660, 1290, 1185, 1080, 970 $cm^{-1}$.

MS: 276 ($M^+$, 1), 220 (2), 207 (25), 190 (14), 175 (8), 151 (7), 134 (53), 123 (6), 108 (63), 91 (28), 79 (12), 69 (100), 55 (10), 43 (44).

Das Expertenpanel bezeichnete das Raucharoma der analog Beispiel 1 hergestellten Testzigaretten als demjenigen der Kontrollzigaretten überlegen, insbesondere weil im ersteren Falle der allgemeine Tabakeindruck verstärkt zur Geltung kam.

Beispiel 22

Analog Beispiel 7 werden aus 2,50 g (0,012 Mol) 3-Oxo-α-ionol, 1,12 g (0,015 Mol) Pyridin und 1,86 g (0,012 Mol) Phenylacetylchlorid 3,60 g (92%) ca. 90%-iges 3-Oxo-α-ionylphenylacetat erhalten.

IR: 1730, 1665, 1630, 1600, 1495, 1250, 1140, 1045, 980, 730, 700 $cm^{-1}$.

MS: 326 ($M^+$, 1), 207 (4), 191 (26), 175 (5), 149 (8), 137 (26), 135 (41), 118 (34), 107 (14), 91 (100), 79 (9), 65 (13), 55 (10), 43 (19).

Das Expertenpanel bezeichnete das Raucharoma der analog Beispiel 1 hergestellten Testzigaretten als süsser und heuartiger als dasjenige der Kontrollzigaretten.

Beispiel 23

Analog Beispiel 8 werden aus 1,50 g (7,2 mMol) 3-Oxo-α-ionol, 0,63 g (8,7 mMol) Pyridin und 2,13 g (ca. 7,2 mMol) des Säurechlorid-Gemisches ex Leinöl 2,7 g (82%) Produkt erhalten, welches sich aus ca. 57% 3-Oxo-α-linolenat, 14% -linoleat, 18% -oleat, 3% -stearat und 7% -palmitat zusammensetzt.

IR: 1735, 1670, 1635, 1245, 1175, 1045, 970, 720 $cm^{-1}$.

MS: 468 ($M^+$, <1), 277 (35), 207 (15), 192 (100), 177 (24), 149 (25), 137 (68), 135 (94), 123 (34), 108 (38), 91 (49), 83 (58), 67 (57), 55 (82), 43 (55).

Das Expertenpanel bezeichnete das Raucharoma der analog Beispiel 1 hergestellten Testzigaretten als milder als dasjenige der Kontrollzigaretten. Zudem wurde ein angenehmer «Salivating-Effekt» festgestellt.

Beispiel 24

Analog Beispiel 2 werden aus 1,50 g (7,1 mMol) 3-Oxo-dihydro-α-ionol, 1,12 g (14,2 mMol) Pyridin und 0,87 g (8,6 mMol) Essigsäureanhydrid 1,60 g Rohprodukt erhalten, aus denen sich durch Kugelrohrdestillation 1,15 g (65%) ca. 90%-iges 3-Oxo-dihydro-α-ionylacetat gewinnen lassen. Sdp.ca. 115 °C/0,05 mmHg.

IR: 1735, 1665, 1630, 1242, 1132, 1047, 1022, 950 $cm^{-1}$.

NMR: 1.07 und 1.10 (je s, je 3H); 1.27 (d, J~6.5, 3H); 2.05 (s, 3H); 2.07 (s, 3H); 1.50 – 2.62 (m, 7H); 4.90 (m, 1H); 5.84 (s, 1H).

MS: 252 ($M^+$, 3), 209 (1),192 (4), 177 (9), 163 (3), 150 (10), 138 (12), 135 (20) 123 (13), 121 (15), 108 (32), 93 (23), 79 (10), 67 (15), 55 (16), 43 (100).

Das Expertenpanel bezeichnete das Raucharoma der analog Beispiel 1 hergestellten Testzigaretten als abgerundeter als dasjenige der Kontrollzigaretten; zugleich wurde der allgemeine Tabakeindruck verstärkter wahrgenommen.

Beispiel 25

Analog Beispiel 5 werden aus 1,50 g (7,1 mMol) 3-Oxo-dihydro-α-ionol, 1,12 g (14,2 mMol) Pyridin und 1,60 g (8,6 mMol) Valeriansäureanhydrid-1,95 g Rohprodukt erhalten, aus denen sich durch Kugelrohrdestillation 1,44 g (69%) ca. 90%-iges 3-Oxo-dihydro-α-ionylvalerat gewinnen lassen. Sdp.ca. 130 °C/0.05 mmHg.

IR: 1730, 1665, 1630, 1250, 1180, 1132 $cm^{-1}$.

MS: 294 ($M^+$, 16), 209 (30), 192 (27), 177 (45), 163 (16), 150 (55), 138 (59), 135 (88) 123 (52), 12 (49), 108 (100), 93 (64), 85 (55), 79 (28), 67 (46), 57 (75), 41 (80).

Das Expertenpanel bezeichnete das Raucharoma der analog Beispiel 1 hergestellten Testzigaretten als mehr an orientalischen Tabak erinnernd als dasjenige der Kontrollzigaretten.

Beispiel 26

Analog Beispiel 7 werden aus 1,70 g (8,1 mMol) 3-Oxo-dihydro-α-ionol, 7,90 g (10 mMol) Pyridin und 1,25 g (8,1 mMol) Phenylacetylchlorid 2,22 g (84%) ca. 90%-iges 3-Oxo-dihydro-α-ionylphenylacetat erhalten.

IR: 1730, 1665, 1630, 1600, 1495, 1250, 1162, 1130, 1085, 725, 700 $cm^{-1}$.

MS: 328 ($M^+$, 30), 209 (7),193 (56), 177 (16), 163 (8), 151 (34), 135 (42), 118 (39) 109 (58), 91 (100), 79 (14), 67 (31), 55 (22), 41 (25).

Das Expertenpanel bezeichnete das Raucharoma der analog Beispiel 1 hergestellten Testzigaretten als süsser und heuartiger als dasjenige der Kontrollzigaretten.

Beispiel 27

Analog Beispiel 8 werden aus 1,05 g (5,0 mMol) 3-Oxo-dihydro-α-ionol, 0,55 g (7,0 mMol) Pyridin und 1,48 g (ca. 5 mMol) des Säurechlorid-Gemisches ex Leinöl 2,0 g (85%) Produkt erhalten, welches sich aus ca. 57% 3-Oxo-dihydro-α-ionyllionolenat, 14% -linoleat, 18% -oleat, 3% -stearat und 7% -palmitat zusammensetzt.

IR: 1730, 1665, 1630, 1250, 1180, 1130, 725 $cm^{-1}$.

MS: 470 (3), 277 (17), 209 (32), 193 (84), 177 (22), 151 (38), 138 (55), 135 (69) 123 (39), 109 (100), 95 (43), 79 (38), 67 (68), 55 (62), 43 (54).

Das Expertenpanel bezeichnete das Raucharoma der analog Beispiel 1 hergestellten Testzigaretten als milder und ausgewogener als dasjenige der Kontrollzigaretten.

Wie vorne ausgeführt, verbessert ein Zusatz eines Oxo-ionylesters der Formel I zu einem Tabakerzeugnis dessen Rauchcharakteristik deutlich, es wird insbesondere der allgemeine Tabakeindruck verstärkt, und das gesamte Aroma erscheint abgerundeter und voller.

Die Ester der Formel I unterscheiden sich nun in diesem Aspekt ganz deutlich von den Estern der japanischen Patentpublikation Nr. 57734 (Japan Tobacco ans Salt Pub., vom 20. Mai 1981, angemeldet am 16. Oktober 1979) mit der allgemeinen Formel

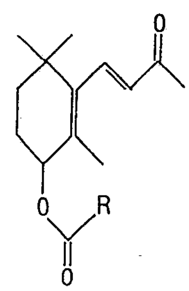

III

Das Formiat, Acetat, Propionat, n-Butyrat, n-Valerianat, 2-Methylbutyrat und das Crotonat,

also R in der Formel III = H, $-CH_3$, $-CH_2CH_3$, $-CH_2CH_2CH_3$, $-(CH_2)_3CH_3$, $-CH{=}CH{-}CH_3$.

Das Expertenpanel bezeichnete das Raucharoma der mit allen diesen bekannten Estern auf die im Beispiel 1 beschriebene Art und Weise aromatisierten Testzigaretten im Vergleich zu den entsprechenden Zigaretten mit Zusatz der erfindungsgemässen Verbindungen I in jedem Fall als eindeutig durch parfümistisch wirkende, stark blumige Noten gekennzeichnet.

Diese (zu) starke Parfümierung der Zigaretten war aber im vorliegenden Fall nicht gesucht, sondern die Zielsetzung war vielmehr, einen vollen, nicht-parfümierten Tabakcharakter im (Rauch)Aroma optimal zur Geltung zu bringen. Diese Zielsetzung ist nun aber für die Vielzahl der Raucher insbesondere im Zeitalter der Zigaretten mit niedrigem Nikotingehalt [light cigarettes, lights, lowdelivery cigarettes, low tar cigarettes], meistens erzielt durch sogenannte High-retention-filter und/oder Ventilation von ausserordentlicher Wichtigkeit , da letztere Techniken bekanntlich das Tabakaroma meistens gewichtig beeinträchtigen.

Die ausgesprochene Parfümierung von Zigaretten – die selbstverständlich bei bestimmten Tabakmischungen nach wie vor ebenfalls durchaus berechtigt und erwünscht sein kann – tritt dabei in den Hintergrund.

**Patentansprüche**

1. Geschmackstoffkomposition, insbesondere zur Verstärkung, Verbesserung, Erhöhung oder Modifizierung der organoleptischen Eigenschaften von Tabakerzeugnissen, gekennzeichnet durch einen Gehalt an einer Verbindung der Formel

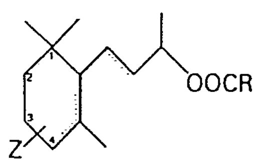

I

worin RCO für einen Acylrest mit 1 – 18 Kohlenstoffatomen steht, eine der gestrichelt gezeichneten Linien im Ring eine zusätzliche Bindung und die gestrichelt gezeichnete Linie in der Seitenkette eine fakultative zusätzliche Bindung bedeutet und Z eine Oxogruppe in α-Stellung zur ringständigen Doppelbindung darstellt.

2. Komposition gemäss Anspruch 1, dadurch gekennzeichnet, dass RCO in der Formel I einen Alkanoyl- oder Alkenoylrest mit bis zu 18 Kohlenstoffatomen darstellt.

3. Komposition gemäss Anspruch 1, dadurch gekennzeichnet, dass sie 4-Oxo-β-ionylvalerat, 4-Oxo-β-ionylisovalerat, 4-Oxo-β-ionylbutyrat, 4-Oxo-β-ionylacetat, 4-Oxo-β-ionylformiat, 4-Oxo-β-ionylcrotonat, 4-Oxo-dihydro-β-ionylvalerat, 4-Oxo-dihydro-β-ionylacetat oder 3-Oxo-α-ionylvalerat enthält.

4. Verfahren zum Verstärken, Verbessern, Erhöhen und Modifizieren der organoleptischen Eigenschaften von Tabakerzeugnissen, dadurch gekennzeichnet, dass man eine Verbindung der Formel

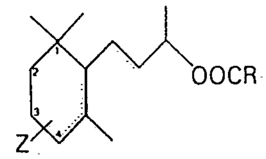

I

worin RCO für einen Acylrest mit 1 – 18 Kohlenstoffatomen steht, eine der gestrichelt gezeichneten Linien im Ring eine zusätzliche Bindung und die gestrichelt gezeichnete Linie in der Seitenkette eine fakultative zusätzliche Bindung bedeutet und Z eine Oxogruppe in α-Stellung zur ringständigen Doppelbindung darstellt, verwendet.

5. Verwendung von Verbindungen der Formel

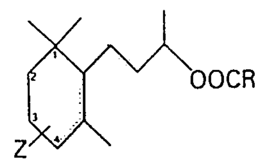

I

worin RCO für einen Acylrest mit 1 – 18 Kohlenstoffatomen steht, eine der gestrichelt gezeichneten Linien im Ring eine zusätzliche Bindung und die gestrichelt gezeichnete Linie in der Seitenkette eine fakultative zusätzliche Bindung bedeutet und Z eine Oxogruppe in α-Stellung zur ringständigen Doppelbindung darstellt, als Geschmackstoffe, insbesondere zur Aromatisierung von Tabak.

6. Verbindungen der Formel

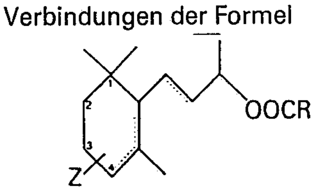

I

worin RCO für einen Acylrest mit 1 – 18 Kohlenstoffatomen steht, eine der gestrichelt gezeichneten Linien im Ring eine zusätzliche Bindung und die gestrichelt gezeichnete Linie in der Seitenkette eine fakultative zusätzliche Bindung bedeutet und Z eine Oxogruppe in α-Stellung zur ringständigen Doppelbindung darstellt, mit Ausnahme der Acetate.

7. Verbindungen der Formel I gemäss Anspruch 6, dadurch gekennzeichnet, dass RCO in der Formel I einen Alkanoyl- oder Alkenoylrest mit bis zu 18 Kohlenstoffatomen darstellt.

8. 4-Oxo-β-ionylformiat.

9. 4-Oxo-β-ionylbutyrat.

10. 4-Oxo-β-ionylvalerat.

11. 3-Oxo-α-ionylvalerat.

12. 4-Oxo-β-ionylisovalerat.

13. 4-Oxo-dihydro-β-ionylvalerat.

14. 4-Oxo-β-ionylcrotonat.

15. Verfahren zur Herstellung von Verbindungen der Formel

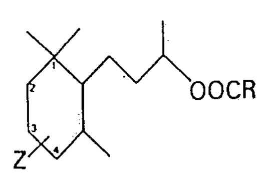

I

worin RCO für einen Acylrest mit 1 – 18 Kohlenstoffatomen steht, eine der gestrichelt gezeichneten Linien im Ring eine zusätzliche Bindung und die gestrichelt gezeichnete Linie in der Seitenkette eine fakultative zusätzliche Bindung bedeutet und Z eine Oxogruppe in α-Stellung zur ringständigen Doppelbindung darstellt, dadurch gekennzeichnet, dass man eine Verbindung der Formel

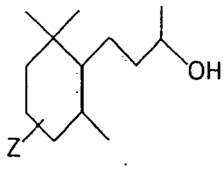

II

worin Z und die gestrichelt gezeichneten Linien obige Bedeutung besitzen, verestert, wobei die Herstellung der Acetate ausgeschlossen sein soll.

**Claims**

1. A flavouring composition, especially for the intensification, improvement, enhancement or modification of the organoleptic properties of tobacco products, characterized by a content of a compound of the formula

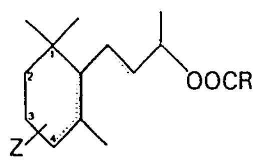

I

wherein RCO stands for an acyl residue with 1 – 18 carbon atoms, one of the dotted lines in the ring signifies an additional bond and the dotted line in the side-chain signifies an optional additional bond and Z represents an oxo group in the α-position to the double bond in the ring.

2. A composition in accordance with claim 1, characterized in that RCO in formula I represents an alkanoyl or alkenoyl residue with up to 18 carbon atoms.

3. A composition in accordance with claim 1, characterized in that it contains 4-oxo-β-ionyl valerate, 4-oxo-β-ionyl isovalerate, 4-oxo-β-ionyl butyrate, 4-oxo-β-ionyl acetate, 4-oxo-β-ionyl formate, 4-oxo-β-ionyl crotonate, 4-oxo-dihydro-β-ionyl valerate, 4-oxo-dihydro-β-ionyl acetate or 3-oxo-α-ionyl valerate.

4. A method for the intensification, improvement enhancement and modification of the organoleptic properties of tobacco products, characterized by using a compound of the formula

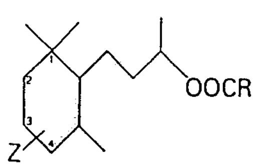

I

wherein RCO stands for an acyl residue with 1 – 18 carbon atoms, one of the dotted lines in the ring signifies an additional bond and the dotted line in the side-chain signifies an optional additional bond and Z represents an oxo group in the α-position to the double bond in the ring.

5. The use of compounds of the formula

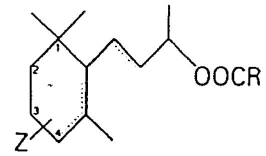

I

wherein RCO stands for an acyl residue with 1 – 18 carbon atoms, one of the dotted lines in the ring signifies an additional bond and the dotted line in the side-chain signifies an optional additional bond and Z represents an oxo group in the α-position to the double bond in the ring, as flavorants, especially for the flavouring of tobacco.

6. Compounds of the formula

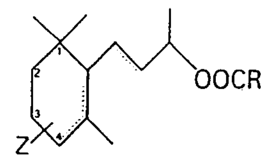

I

wherein RCO stands for an acyl residue with 1 – 18 carbon atoms, one of the dotted lines in the ring signifies an additional bond and the dotted line in the side-chain signifies an optional additional bond and Z represents an oxo group in the α-position to the double bond in the ring, with the exception of the acetates.

7. Compounds of formula I in accordance with claim 6, characterized in that RCO in formula I represents an alkanoyl or alkenoyl residue with up to 18 carbon atoms.

8. 4-Oxo-β-ionyl formate.

9. 4-Oxo-β-ionyl butyrate.

10. 4-Oxo-β-ionyl valerate.

11. 3-Oxo-α-ionyl valerate.

12. 4-Oxo-β-ionyl isovalerate.

13. 4-Oxo-dihydro-β-ionyl valerate.

14. 4-Oxo-β-ionyl crotonate.

15. A process for the manufacture of compounds of the formula

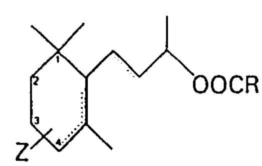

I

wherein RCO stands for an acyl residue with 1 – 18 carbon atoms, one of the dotted lines in the ring signifies an additional bond and the dotted line in the side-chain signifies an optional additional bond and Z represents an oxo group in the α-position to the double bond in the ring, characterized by esterifying a compound of the formula

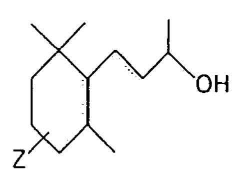

II

wherein Z and the dotted lines have the above significance, whereby the manufacture of the acetates is excluded.

## Revendications

1. Composition d'arôme, convenant en particulier pour le renforcement, l'amélioration, l'accentuation ou la modification des propriétés organoleptiques des produits de tabac, caractérisée en ce qu'elle contient un composé de formule

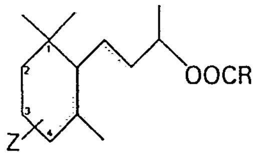

I

dans laquelle RCO représente un radical acyle en C 1 – 18, l'un des traits en pointillé du cycle représente une liaison supplémentaire et le trait en pointillé de la chaîne latérale une liaison supplémentaire facultative, et Z représente un groupe oxo en position α de la double liaison cyclique.

2. Composition selon la revendication 1, caractérisée en ce que, dans la formule I, RCO représente un radical alcanoyle ou alcénoyle contenant jusqu'à 18 atomes de carbone.

3. Composition selon la revendication 1, caractérisée en ce qu'elle contient du valérate de 4-oxo-β-ionyle, de l'isovalérate de 4-oxo-β-ionyle, du butyrate de 4-oxo-β ionyle, de l'acétate de 4-oxo-β-ionyle, du formiate de 4-oxo-β-ionyle, du crotonate de 4-oxo-β-ionyle, du valérate de 4-oxo-dihydro-β-ionyle, de l'acétate de 4-oxo-dihydro-β-ionyle ou du valérate de 3-oxo-α-ionyle.

4. Procédé pour renforcer, améliorer, accentuer et modifier les propriétés organoleptiques des produits de tabac, caractérisé en ce que l'on utilise un composé de formule

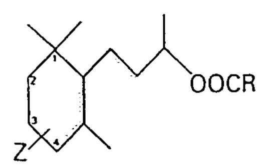

I

dans laquelle RCO représente un radical acyle en C 1 – 18, l'un des traits en pointillé du cycle représente une liaison supplémentaire et le trait en pointillé de la chaîne latérale représente une liaison supplémentaire facultative, et Z représente un groupe oxo en position α de la double liaison cyclique.

5. Utilisation de composés de formule

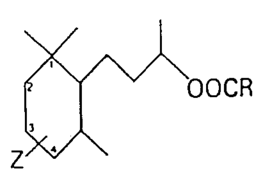

I

dans laquelle RCO représente un radical acyle en C 1 – 18, l'un des traits en pointillé du cycle représente une liaison supplémentaire et le trait en pointillé de la chaîne latérale représente une liaison supplémentaire facultative, et Z représente un groupe oxo en position α de la double liaison cyclique, en tant qu'arômes, en particulier pour l'aromatisation du tabac.

6. Composés de formule

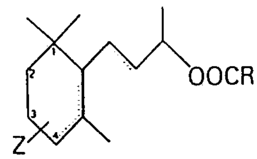

I

dans laquelle RCO représente un radical acyle en C 1– 18, l'un des traits en pointillé du cycle représente une liaison supplémentaire et le trait en pointillé de la chaîne latérale représente une liaison supplémentaire facultative, et Z représente un groupe oxo en position α de la double liaison cyclique, à l'exception des acétates.

7. Composés de formule I selon la revendication 6, caractérisés en ce que, dans la formule I, RCO représente un groupe alcanoyle ou alcénoyle contenant jusqu'à 18 atomes de carbone.

8. Le formiate de 4-oxo-β-ionyle.

9. Le butyrate de 4-oxo-β-ionyle.

10. Le valérate de 4-oxo-β-ionyle.

11. Le valérate de 3-oxo-α-ionyle.

12. L'isovalérate de 4-oxo-β-ionyle.

13. Le valérate de 4-oxo-dihydro-β-ionyle.

14. Le crotonante de 4-oxo-β-ionyle.

15. Procédé de préparation des composés de formule

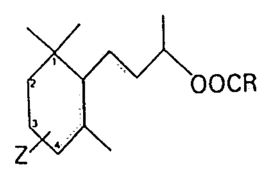

I

dans laquelle RCO représente un radical acyle en C 1 – 18, l'un des traits en pointillé du cycle représente une liaison supplémentaire et le trait en pointillé de la chaîne latérale représente une liaison supplémentaire facultative, et Z représente un groupe oxo en position α de la double liaison cyclique, caractérisé en ce que l'on estérifie un composé de formule

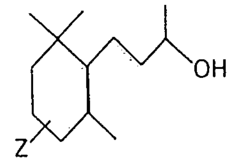

II

dans laquelle Z et les traits en pointillé ont les significations indiquées ci-dessus, la préparation des acétates étant exclue.